(19) Europäisches Patentamt
European Patent Office
Office européen des brevets

(11) Publication number: 0 137 515
A2

# (12) EUROPEAN PATENT APPLICATION

(21) Application number: 84112326.8

(22) Date of filing: 12.10.84

(51) Int. Cl.[4]: G 01 N 33/533
G 01 N 33/542, G 01 N 33/58
G 01 N 33/74, C 12 Q 1/68

(30) Priority: 13.10.83 US 541405

(43) Date of publication of application:
17.04.85 Bulletin 85/16

(84) Designated Contracting States:
AT BE CH DE FR GB IT LI NL SE

(71) Applicant: UNIVERSITY OF GEORGIA RESEARCH FOUNDATION, INC.
612 Boyd Graduate Studies Research Center
Athens, GA 30602(US)

(72) Inventor: Cormier, Milton J.
Route 1 Box 34A
Bogart Georgia 30622(US)

(72) Inventor: Patel, Ashokkumar
1907 South Milledge Avenue Apt. B-6
Athens Georgia 30605(US)

(74) Representative: Lederer, Franz, Dr. et al,
Patentanwälte Dr. Lederer Franz Meyer-Roxlau Reiner F.
Lucile-Grahn-Strasse 22
D-8000 München 80(DE)

## (54) Bioluminescent immunoassays.

(57) A process for detecting ligand-ligand interactions such as antigen-antibody, hapten-antibody, hormone-receptor, protein-nucleic acid, and nucleic acid-nucleic acid utilizing a coelenterate-derived luciferase or a coelenterate-derived photoprotein which is a bioluminescent, low molecular weight, single polypeptide chain as a label in a bioluminescence-linked immunoassay is disclosed. The disclosed process is rapid and process provides greater sensitivity over existing processes. Also, the disclosed process utilizes labels and reagents which possess a shelf life of years. Three types of bioluminescence-lined immunoassays are disclosed: heterogeneous assay requiring a single separation step, homogeneous assay without separation step requiring dual wavelength measurements, and a homogeneous assay without a separation step requiring a single wavelength measurement.

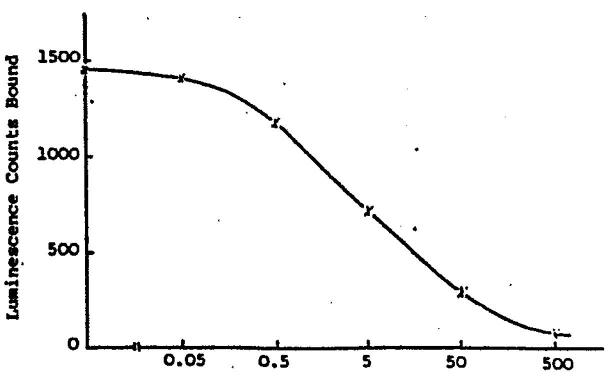

Figure 2



Croydon Printing Company Ltd.

Ref UG 4093/71

# Bioluminescent Immunoassays

Immunoassays are now widely used in clinical medicine for diagnostic purposes. A wide variety of molecules found in biological fluids, such as proteins, hormones, viruses, and exogenous drugs, may be quantitatively assayed by these techniques. The basis of all of these methods is the specific recognition of antigens by antibodies.

Radioimmunoassays (RIA), first developed by Yalow and Berson in 1960, use radioisotopes as lables and are still widely used. As illustrated in equation (1), below, RIA is based on a competition between unlabelled antigen (Ag) and labelled antigen (Ag*) for a limited number of antibody (Ab) binding sites resulting in the partitioning of the label between free and bound states. Either the free or bound labelled antigen may be measured allowing quantitation of the antigen. RIA, or a competitive binding assay, is illustrated as shown in equation (1):

$$\begin{array}{ccc} Ag^* + Ab & \rightleftharpoons & Ag^*\text{-}Ab \\ \quad\upharpoonleft\downharpoonright Ag & & \\ Ag\text{-}Ab & & \end{array} \qquad (1).$$

Although RIA is broadly applicable, is highly sensitive and specific, the method also has substantial disadvantages. These include the requirement that a separation step be employed for free and bound labels, isotope-related health hazards, disposal of radioisotopes, the use of expensive equipment, and the short shelf life of isotopically-labelled reagents. Usually the label is $^{125}I$ and the shelf life of $^{125}I$-labelled reagents is on the order

of a few weeks.

Because of the disadavantages of the RIA outlined above, immunoassays based on non-isotopic labels have been developed over the past decade. Among the more widely used examples are the enzymeimmunoassays (EIA) in which an enzyme, rather than a radioisotope, is linked to an antigen. There are two types of EIA, heterogeneous and homogeneous. An assay which requires separation of the free and bound labelled antigen (as in RIA) is termed a heterogenous assay. An assay which does not require such a separation step, due to modulation of the properties of the indicator molecule by the antigen-antibody reaction, is referred to as a homogeneous assay.

The heterogeneous EIA is an enzyme-linked immunosorbent assay (ELISA) as exemplified by United States Patent No. 3,654,090 and United States Patent No. 3,791,932. In ELISA, the antibody is usually attached to a solid support to simplify the separation of free and bound label. The enzymes most commonly used as labels are horseradish peroxidase, glucose oxidase, ß-galactosidase, and alkaline phosphatase.

The homogeneous EIA is an enzyme multiplied immunoassay (EMIT) as exemplified by United States Patent No. 3,817,837. EMIT is based on the principle that the properties of bound and free antigen-enzyme are different. Because of this a separation step is not required in order to determine the concentration of antigen in the sample. The most commonly used enzymes whose activities are altered by ligand-specific antibodies are lysozyme, malate dehydrogenase, and glucose-6-phosphate dehydrogenase.

Examples of both ELISA's and EMIT's are discussed in "Diagnostic Immunology" (W. Keitges and M. Nakamura, eds) College of American Pathologists, Skokie, Ill., 1980. These

assays have some advantages over RIA's since enzymes, rather than radioisotopes, are used as labels resulting in longer shelf lifes of reagents. EMIT has the added advantage of not requiring a separation step. The disadvantages of the assays are based on the inherent sensitivity of enzymes of their environment and the wide variation of catalytic activity of enzymes. That is, the enzymes used as labels must be carefully selected for their high catalytic activity in order to provide the sensitivity required in the assay. Furthermore, the inherent instability of enzymes which may result from environmental changes make it difficult to choose coupling procedures which do not destroy most of the enzyme activity. Thus these assays are severely restricted in their use and are generally applicable only to low molecular weight compounds.

In addition to the enzyme-labels outlined above, fluorescent molecules have also been used in immunoassays as replacements for radioisotope-labels as exemplified by French Patent No. 2,217,350. Fluorescence polarization techniques were first adapted to immunoassays by Dandliker et al. (Immunochem. 1, 165 (1964); Immunochem. 10, 219 (1973). The principle involves an observed increase in the fluorescence polarization of a low molecular weight fluorescence molecule when it becomes bound to a large molecular weight species (antibody). Since the fluorescence polarization is modulated upon binding, a homogeneous immunoassay is possible. Ullman et al. (J. Biol. Chem. 251, 4172 (1976) have utilized the principle of fluorescence excitation transfer to detect antigen-antibody complex formation. Generally, a fluorescent-labelled antigen, acting as energy donor, and an antibody labelled with a second fluorescent--label, acting as energy acceptor, forms the basis of a homogeneous immunoassay. Since the efficiency of energy transfer between donor and acceptor molecules is inversely related to the sixth power of the distance between them, energy transfer occurs only upon complex formation thus

eliminating the need for a separation step. These assays also have restricted usages since the positioning of the label in the antibody is important in order to achieve energy transfer. Secondly, since fluorescent molecules vary widely in their quantum yields of fluorescence, only those fluorescent molecules which have reasonably high quantum yields are useful. Generally, these fluorescence-based immunoassays are considerably less sensitive than their RIA counterparts and their usefulness is further restricted for this reason.

More recently, immunoassays have been developed which employ chemiluminescent compounds as labels. A chemiluminescent compound is one which, under appropriate conditions, can be oxidized to produce a fluorescence product in the singlet electronic excited state. Decay to the ground state produces a photon of light. Examples are luminol, isoluminol, biacridinium salts, lophine, and oxalate esters. Since the emission of visible light is easily measured and quantitated, chemiluminescent labels have been successfully used in certain types of immunoassays. Chemiluminescent immunoassays (CLIA) usually involve coupling a chemiluminescent compound to an antigen and measuring light, rather than radioactivity, as the end point. Examples of chemiluminescent assays are disclosed in United States Patent No. 4,220,450, United States Patent No. 4,104,029, and United Kingdom Patent No. 1,461,877. Recent progress in the field of chemiluminescent immunoassays is also summarized in "Luminescent Assays" (M. Serio and M. Pazzagli, eds.) Vol. 1, Raven Press, 1982.

Homogeneous chemiluminescent immunoassays have recently been described based on energy transfer (Patel et al., Biochem., Soc. Trans. 11, 196 (1983). Aminobutylethyl-isoluminol (ABEI) was coupled to an antigen as label and the antibody was labelled with an energy transfer acceptor, a fluorescent molecule such as fluorescein. When the ABEI--

labelled antigens bind to their respective fluorescein--labelled antibodies a shift is observed in the ratio of chemiluminescence at 460 nm (blue) to that at 525 nm (Green). This energy transfer process was used to develop homogeneous CLIA assays for several antigens.

The sensitivity of CLIA's is limited by the quantum yields of chemiluminescence. The quantum yields of such compounds, in the aqueous environments used for immunoassays, are generally very low (ι0.1%); however, a few labels, such as ABEI discussed above, provide sensitivities for CLIA's comparable to those observed for RIA's.

The present invention utilizes bioluminescent proteins, specifically coelenterate-derived photoproteins and coelenterate-derived luciferases, as labels in contrast to the chemiluminescent labels described above. These bioluminescent protein labels can be utilized in the detection of ligand-ligand interactions generally. Examples of such ligand-ligand interactions are interactions between antigen-antibody, hapten-antibody, hormone-receptor, protein--nucleic acid, nucleic acid-nucleic acid, and the like. Examples of ligand-ligand interactions are disclosed below; however, such ligand-ligand interactions are not intended to limit the scope of this invention.

Bioluminescence-linked luminescent immunoassays (BLIA) differ from CLIA wherein chemiluminescent labels are used. With one exception, bioluminescent proteins have not previously been employed as labels in luminescent immunoassays, although firefly and bacterial luciferases have been used to detect their respective substrates (such as ATP and NADH) which have been utilized as labels in certain cases. Examples of the uses of firefly and bacterial luciferases to detect their respective substrates are disclosed in United States Patent No. 1,522,607 and in a recent book (Luminescent Assays, M. Serio and M. Pazzagli, eds., Vol.

1; Raven Press, 1982, pp. 57, 89, and 115). In such recent book (Luminescent Assays, M. Serio and M. Pazzagli, eds., Vol. 1, Raven Press, 1982, pp. 115 and 125) examples are given in which firefly luciferase is used as a label in heterogeneous immunoassays for the detection of picomol quantities of methotrexate and trinitrotoluene which is the one exception referenced above.

As indicated above, there has been one report on the use of firefly luciferase as a label in a heterogeneous immunoassay. However, there are several problems associated with the use of this protein as a label. Firstly, chemical markets cannot rely on using firefly luciferase as a label since the supply of this luciferase depends on the field collection of fireflys whose population fluctuates widely from year to year. Secondly, firefly bioluminescence requires ATP for light emission and since ATP occurs in all biological tissues and fluids an undesirable background would exist in any rapid, homogeneous BLIA based on firefly luciferase. Components of the coelenterate-derived bioluminescence system are confined to coelenterates and would not pose this type of background problem. Thirdly, energy transfer processes are not components of the firefly system; and it is, therefore, not possible to develop homogeneous immunoassays with firefly luciferase based on this principle.

The use of coelenterate-derived proteins as labels in immunoassays would circumvent the problems outlined above. For example, the coelenterate proteins are low molecular weight, single polypeptide proteins in contrast to the multiple polypeptide chain and relatively high molecular weight firefly luciferase. This difference produces (1) increased stability of the label during the coupling procedures; and (2) unlimited supply of such proteins can be more readily prepared utilizing recombinant DNA technology known in the art. In addition, homogeneous (as well as

heterogeneous) immunoassays can be developed using coelenterate-derived luciferases and photoproteins because of energy transfer processes that occur naturally in these systems. Furthermore, the recharging of apophotoproteins can be carried out with a variety of analogues of coelenterate luciferin (Formula 1) and could lead to the development of novel homogeneous BLIA. Finally, the terms luciferase and luciferin are generic terms. Thus coelenterate, firefly and bacterial luciferases and luciferins are distinct molecular entities and bear no known similarities to one another.

A number of features of the present invention make it attractive for use in clinical medicine. As indicated in some of the examples below, the labels are stable when coupled to ligands or antibodies. Thus the shelf life would be on the order of months to years as compared to RIA where the shelf life of labelled reagents is on the order of weeks. The labels used in this invention result in the production of bioluminescence whose quantum yields are relatively high (ι15%). This provides sensitivities on the order of femtomoles of ligand. Generally, the sensitivities of the assays (BLIA) of the present invention, are equal to or greater than other currently used immunoassays. Light production is instantaneous making the assays rapid. The measurement of light is not only rapid and sensitive, but the commercial instruments available are inexpensive wherein smaller hospitals and clinics can perform BLIA's. The reagents for BLIA's are relatively inexpensive.

It is an object of the present invention to utilize bioluminescent, low molecular weight single polypeptide chain proteins as a label in detecting ligand-ligand interactions such as antigen-antibody, hapten-antibody, hormone-receptor, protein-nucleic acid, and nucleic acid-nucleic acid. Also, it is an object of the present invention to utilize coelenterate-derived photoproteins and luciferases as labels to develop heterogeneous and homogeneous bioluminescent immunoassays (BLIA's). Additionally, it is an object of the present invention to use naturally occurring, coelenterate-derived and synthetic energy transfer acceptors to develop homogeneous BLIA's. It is a further object to use analogues of coelenterate luciferin in order to develop homogeneous BLIA's based on single wavelength determinations. In addition, it is an object of the present invention to use apophotoproteins as labels in BLIA's by recharging such proteins in the presence of coelenterate luciferin or one of its synthetic analogues.

These and other objects, aspects, and advantages of this invention will become apparent from the considerations given below.

## Brief Description of the Drawings

Figure 1 is the progesterone-aequorin antibody dilution curve which shows the luminescence counts bound to the solid phase antibodies at various dilutions. Each point shows the mean standard deviation of triplicate determinations. Figure 2 is the progesterone BLIA standard curve which shows the luminescent counts bound to the solid phase antibodies at varying progesterone concentrations. Figure 3 is the rabbit IgG-aequorin conjugate binding curve which shows the luminescence counts (or rabbit IgG-aequorin conjugate) bound to the varying antibody dilutions.

The present invention is a process for detecting a ligand-ligand interaction utilizing a bioluminescent, low molecular weight, single polypeptide chain protein as a label in a bioluminescence-linked immunoassay. Representative ligand-ligand interactions of the present invention are antigen-antibody, hapten-antibody, hormone-receptor, protein-nucleic acid, and nucleic acid-nucleic acid. The ligand being assayed in the present invention is a molecule having a specific antibody capable of being produced

against such molecule. The label is a coelenterate-derived luciferase or a coelenterate-derived photoprotein. The anthozoan coelenterates, such as Renilla, Cavernularia, Ptilosarcus, and Stylatula, contain a common enzyme (luciferase) which catalyzes the bioluminescent oxidation of a chromophore (luciferin) common to all of the bioluminescent coelenterates (M. J. Cormier (1981) in "Bioluminescence and Chemiluminescence" (M. DeLuca and W. McElroy, eds), Academic Press, pp. 225-233; M. J. Cormier (1978) in Bioluminescence in Action (P. Herring, ed.) Academic Press, pp. 75-108).

Coelenterate luciferin has the structure shown in Formula 1, below, and has been isolated from a variety of species of coelenterates (M. J. Cormier, et al. (1973) J. Cell. Physiol. 81, 291; W. Ward and M. J. Cormier (1975) Proc. Natl. Acad. Sci., 72, 2530; K. Hori, et al. (1977) Proc. Natl. Acad. Sci., 74, 4285). Coelenterate luciferin is disclosed in formula (1):

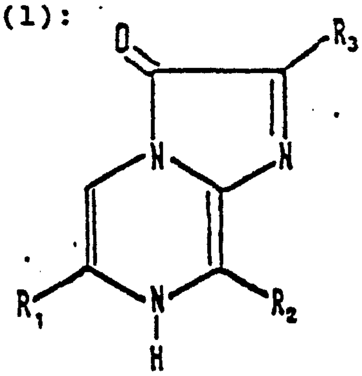

wherein $R_1$ is a p-hydroxyphenyl group, wherein $R_2$ is a benzyl group, and wherein $R_3$ is a p-hydroxybenzyl group. The present invention utilizes the specific coelenterate luciferin described above as a bioluminescence factor; however, the present invention also, utilizes any luciferin-like compound as a bioluminescence factor as shown in formula (1), above, wherein $R_1$ is selected from the group consisting of an aromatic group, a heterocyclic group, an alkyl group and hydrogen, wherein $R_2$ is selected from the group consisting of an aromatic group, a heterocyclic group, an alkyl group, and hydrogen, and wherein $R_3$ is

selected from the group consisting of an aromatic group, a heterocyclic group, an alkyl group, and hydrogen. The aromatic group of any such luciferin-like compound can be selected from the group consisting of a p-hydroxyphenyl group, a benzyl group, and a p-hydroxybenzyl group. When compared with the coelenterate luciferin described above, any such luciferin-like compound may have a different quantum yield or color of light emission or both.

Coelenterate-derived luciferase catalyze the production of blue light ($\varphi_{max}$ = 480 nm) as a result of the chemical oxidation of coelenterate luciferin by dissolved oxygen. The products are oxyluciferin, $CO_2$ and light as shown in equation (2):

$$\text{luciferin} + O_2 \xrightarrow{\text{luciferase}} \text{oxyluciferin} + CO_2 + \text{light} \qquad (2).$$

Coelenterate-derived photoproteins represent a second type of label to be used in this invention. Photoproteins catalyze the same reaction shown in equation (2), above, (W. Ward and M. J. Cormier (1975) Proc. Natl. Acad. Sci. 72, 2530-2534). Examples of such coelenterate-derived proteins are aequorin, obelin, mnemiopsin and berovin (W. Ward and M. J. Cormier (1975) Proc. Natl. Acad. Sci., 72, 2530-2534. Such coelenterate-derived proteins are stable in the absence of free $Ca^{2+}$ and contain both coelenterate luciferin and $O_2$ bound to the protein. When $Ca^{2+}$, a bioluminescence factor when utilizing coelenterate-derived photoproteins, is added to solutions of such coelenterate-derived proteins blue light is produced as shown in equation (3):

$$\text{photoprotein} + Ca^{2+} \longrightarrow \text{oxyluciferin} + CO_2 + \text{light } (\lambda_{max} = 469 \text{ nm}) \qquad (3).$$

The reaction of equation (3) is referred to as $Ca^{+2}$--triggered photoprotein bioluminescence. One of the products, oxyluciferin, remains bound to the spent photoprotein but it can be removed from the protein. The discharged photoprotein (apophotoprotein) can then be recharged in the absence of $Ca^{2+}$. Also, the discharged photoprotein (apophotoprotein) can be recharged in the presence of synthetic coelenterate luciferin as shown in formula (1), above, or any such other luciferin, dissolved $O_2$, and 2-mercaptoethanol (M. J. Cormier (1981) in "Bioluminescence and Chemiluminescence", M. DeLuca and W. McElroy, eds., Academic Press, pp. 225-233). Recharging of apophotoproteins in the absence of $Ca^{2+}$ is shown in equation (4):

$$\text{apophotoprotein} + \text{luciferin} + O_2 + \text{2-mercaptoethanol} \longrightarrow \text{photoprotein} \quad (4).$$

The addition of $Ca^{2+}$ to the recharged apophotoprotein, as shown in equation (4), above, results in light emission. The cycle of equations (3) and (4), above, can be repeated numerous times.

For the purpose of the present invention, ligands may be any molecule found in biological fluids or tissues against which a specific antibody may be produced and which may be covalently attached to a coelenterate-derived luciferase or photoprotein. Examples of such ligands are hormones (steroid or polypeptide derived); proteins such as enzymes and antibodies, bacteria, e.g. Neisseria gonorrhoea; vitamins; metabolites; pesticides; pheromones; carbohydrates; lipids; nucleic acids; and pharmacologically important compounds such as the opiates, the digitalis glycosides, e.g. digoxin, the barbiturates, cocaine, marijuana, the phenothiazines; tumor antigens, e.g. CEA, and the like. The present invention assays for such ligands. Such examples simply illustrate the present invention.

A. Heterogeneous BLIA.

A competitive binding assay is established by covalently coupling luciferase to a particular antigen in equation (5):

$$\begin{array}{ccc} \text{Ag-Luciferase} + \text{Ab} & \rightleftharpoons & \text{Ab-Ag-Luciferase} \\ \uparrow\downarrow \text{Ag} & & \\ \text{Ab-Ag} & & \end{array} \quad (5).$$

The competitive binding BLIA utilizing coelenterate luciferase as shown in equation (5), above, is analogous to the RIA of equation (1), above. In such assay the antibody (Ab) is attached to a solid support resulting in a rapid one-step separation. The amount of luciferase-labelled antigen (ligand-label) found in the supernatant or pellet is determined simply by the addition of coelenterate luciferin (bioluminescence factor) as shown in formula (1), above, or any such luciferin-like compound, followed by the measurement of peak light intensity or total light per unit time. Chemical synthesis methods have been utilized to synthesize luciferin and luciferin-like compounds (K. Hori and M. J. Cormier (1973) Proc. Natl. Acad. Sci. 70, 120-123; K. Hori, et al. (1973) Biochemistry 12, 4463-4468; Inoue, et al. (1975) Chem. Lett. 141; R. Hart, et al. (1979) Biochemistry 18, 2204-2210). The above-referenced chemical synthesis methods are incorporated herein by reference. More specifically, the process of the present invention as shown in equation (5), above, comprises the following steps: (a) reacting the ligand-label with the ligand being assayed and the specific antibody in a reaction mixture to form an antibody-ligand-label and an antibody-ligand; (b) separating the specific antibody from the reaction mixture by attaching the specific antibody to a solid support; (c) adding a bioluminescence factor to the reaction mixture; (d) measuring light; and (e) determining amount of ligand--

label based on light.

A competitive binding assay has been established by covalently coupling a coelenterate-derived photoprotein such as aequorin to an antigen as shown in equation (6):

$$\begin{array}{ccc} \text{Ag-Photoprotein} + \text{Ab} & \rightleftharpoons & \text{Ab-Ag-photoprotein} \\ \uparrow\downarrow \text{Ag} & & \\ \text{Ab-Ag} & & \end{array} \quad (6).$$

The antibody (Ab) is attached to a solid support and the amount of photoprotein-labelled antigen found in the supernatant or pellet was determined simply by the addition of $Ca^{2+}$ to the mixture followed by the measurement of total light per unit time or peak light intensity. More specifically, the label is a coelenterate-derived photoprotein having a compound bound to the coelenterate-derived photoprotein. Such compound is a coelenterate luciferin or a luciferin-like compound. The antigen being assayed can be covalently coupled to an apophotoprotein by covalent coupling means, and the apophotoprotein can then the recharged by a coelenterate luciferin or a luciferin-like compound.

The native photoprotein has been coupled to antigens or antibodies by using standard protein-protein or protein--hapten coupling procedures (M. O'Sullivan and V. Marks (1981) Methods in Enz., 73, 147-166; B. Erlanger (1980) Methods in Enz., 70, 85-104). The above-referenced coupling procedures are incorporated herein by reference. See B. Homogeneous BLIA, below. Any coupling procedure which maintains the luciferase or photoprotein in its functional form will suffice for the purpose of this invention. Alternatively, the more chemically stable apophotoprotein may be covalently coupled to a hapten or protein followed by the luciferin-dependent recharging of the apophotoprotein as shown in equation (4), above. Although these assays are heterogeneous, i.e., they involve a separation step, they

provide distinct advantages over RIA's. The labelled reagents have an infinite shelf-life, lower detection limits and require very short counting time (ι5 seconds).

B. Homogeneous BLIA.

Homogeneous BLIA, utilizing coelenterate luciferase--labelled antigens, are possible. Although the coelenterate luciferin-luciferase reaction described above produces blue light ($\lambda_{max}$ = 480 nm), which has a broad spectral emission, an examination of Anthozoan bioluminescence in vivo reveals that these organisms produce green light ($\lambda_{max}$ = 509 nm) which has a narrow spectral emission (J. Wampler, et al. (1971) Biochemistry 10, 2903-2909). The difference between the in vivo and in vitro emissions is due to the fact that bioluminescence in the Anthozoans is a sensitized process. The sensitizer (energy transfer receptor) is a protein, termed the green fluorescent protein (GFP) which has been isolated and characterized (W. Ward and M. J. Cormier (1979) J. Biol. Chem. 254, 781-790).

The fluorescence of GPF is identical to the green in vivo luminescence of the Anthozoans such as Renilla. Additionally, the green in vivo emissions can be duplicated in vitro by the addition of 0.1 micromolar concentrations of GFP to a luciferin-luciferase reaction mixture. A shift in spectral distribution occurs, from a broad blue to a narrow green emission. Such spectral shift is due to a non-radiative energy transfer process as evidenced by the fact that the quantum yield for coelenterate luciferin as shown in formula (1), above, oxidation increases several-fold. By the simultaneous measurement of light emission at 480 nm and at 509 nm large changes in the ratio 480/509 nm are observed upon the addition of GFP to a luciferin-luciferase reaction mixture. For the above reasons an example of a homogeneous BLIA is shown in equation (7):

$$\text{Ag-Luciferase} + \underset{\substack{\Big\uparrow\!\!\Big\downarrow\ \text{Ag} \\ \text{Ag-Ab-GFP}}}{\text{Ab-GFP}} \rightleftharpoons \text{Luciferase-Ag-Ab-GFP} \qquad (7).$$

The foregoing homogeneous BLIA utilizes coelenterate luciferase and GFP. In equation (7), above, the ligand (antigen) is covalently coupled to luciferase while GFP is coupled to the antibody. Upon the addition of coelenterate luciferin (formula (1), above) a certain 480/509 nm ratio is observed due to energy transfer. As the amount of ligand (antigen) to be analyzed increases in the reaction mixture, the ratio 480/509 nm also increases. No separation step is required thus providing a homogeneous assay. In equation (7), above, the ligand (antigen) may also be coupled to GFP while the antibody is coupled to luciferase. Furthermore, GFP represents only one example of a suitable energy transfer acceptor. Any fluorescent molecule, whose spectral characteristics are suitable as an energy transfer acceptor, such as fluorescein, phycoerythrin, rhodamine, lucifer yellow dyes, 9,10-bisphenethyuyl anthracene, or the like, may be utilized.

For the purpose of the present invention, the utilization of any such luciferin-like compound in the homogeneous BLIA based on signal enhancement provides distinct advantages. A number of other such luciferin-like compound produce little or no bioluminescence due to quenching of the excited states produced (R. Hart, et al. (1979) Biochemistry <u>18</u>, 2204-2210). A few examples of luciferin-like compounds are benzyl luciferin, chlorophenyl luciferin, and phenyl luciferin. However, the addition of GFP results in over a 200-fold increase in the intensity of bioluminescence wherein case a homogeneous BLIA can be established simply by monitoring light emission at 509 nm in response to increasing amounts of the ligand. In equation (7), above, utilizing, analogues of coelenterate luciferin, the emission at 509 nm would decrease as the concentration of

ligand (Ag) increases. Utilization of such other luciferins provides the basis for homogeneous BLIA assays without the need for simultaneous dual wavelength measurements.

Homogeneous BLIA utilizing coelenterate-derived photoproteins is analogous to assays described above in which a $Ca^{2+}$-triggered photoprotein would replace luciferase as a label. Energy transfer during photoprotein bioluminescence does occur from photoprotein-derived excited states to GFP under appropriate conditions (H. Morise, et al. (1974) Biochemistry 13, 2656-2662). The color of light changes from blue ($\lambda_{max}$ = 469 nm) to green ($\lambda_{max}$ = 509 nm). An example of such homogeneous BLIA utilizing such other luciferins (coelenterate-derived photoproteins) and GFP is shown in equation (8):

$$\begin{array}{ccc} \text{Ag-photoprotein + Ab-GFP} & \rightleftharpoons & \text{photoprotein-Ag-Ab-GFP} \\ \uparrow\downarrow \text{Ag} & & \\ \text{Ag-Ab-GFP} & & \end{array} \quad (8).$$

In equation (8), above, the ligand (antigen) is covalently coupled to a photoprotein while GFP or a suitable fluorescent energy transfer acceptor such as fluorescein is coupled to the antibody. Upon the addition of $Ca^{2+}$ a certain 469/509 nm ratio is observed due to energy transfer. As the amount of ligand to be analyzed increases in the reaction mixture, the ratio 469/509 nm also increases. No separation step is required. Alternatively, the ligand may be coupled to GFP while the photoprotein is coupled to antibody.

Apophotoproteins may also be recharged as shown in equation (4), above, by such other luciferins resulting in altered quantum yields of bioluminescence (R. McCann, et al. (1978) Am. Soc. Photobiol. Abst. p. 54). As in the case of using luciferase as label, such other luciferins provide the basis for homogeneous BLIA by simply monitoring light emission at a single wavelength, such as 509 nm in the case

of GFP. The wavelength of light to be monitored varys depending on the energy transfer acceptor used. Any fluorescent molecule such as fluorescein, phycoerythrin, rhodamine, lucifer yellow dyes, 9,10-bisphenethyuyl anthracene, or the like, whose spectral characteristics are suitable as an energy transfer acceptor may be utilized.

The present invention discloses a homogeneous bioluminescent assay having dual wavelength measurements without the requirement of a separation step. The bioluminescence factor of the above process has the ability to induce a measurable light emission from the coelenterate-derived luciferase. More specifically, this process comprises the following steps: (a) covalently coupling the specific antibody to an energy transfer acceptor by covalent coupling means to form an antibody-energy transfer acceptor; (b) reacting the ligand label with the antibody-energy transfer acceptor and the ligand being assayed in a reaction mixture to form a label-ligand-antibody-energy transfer acceptor and a ligand-antibody-energy transfer acceptor; (c) adding a bioluminescence factor to the reaction mixture; (d) measuring light simultaneously at more than one wavelength to determine a ratio; and (e) determining amount of the ligand being assayed based on the ratio.

Alternatively, the above processes comprise the following steps: (a) covalently coupling the specific antibody to the label by covalent coupling means to form an antibody-label; (b) covalently coupling the ligand being assayed to an energy transfer acceptor by covalent coupling means to form a ligand-energy transfer acceptor; (c) reacting the antibody-label with the ligand-energy transfer acceptor and the ligand being assayed in a reaction mixture to form a label-antibody-ligand-energy transfer acceptor and a ligand-antibody-label; (d) adding a bioluminescence factor to the reaction mixture; (e) measuring light simultaneously at more than one wavelength to determine a ratio; and (f) determining amount of the ligand being assayed based on the ratio. In the above processes when the coelenterate-derived photoprotein alternative is used, a compund having the ability to induce a measurable light emission from the coelenterate-derived photoprotein is bound to the coelenterate-derived photoprotein resulting in dual light emission upon the addition of the bioluminescence factor. The above processes of a homogeneous bioluminescent immunoassay can be modified to provide an assay having a single wavelength measurement as follows: (1) when the coelenterate-derived luciferase system is used the bioluminescence factor has the ability to quench measurable light emission from the coelenterate-derived luciferase; and (2) when the coelenterate-derived photoprotein system is used a compound having the ability to quench measurable light emission from the coelenterate-derived photoprotein is bound to the coelenterate-derived photoprotein.

## Example 1

Synthesis of Progesterone-aequorin Conjugate.

To 5 mg of progesterone-11α-hemisuccinate in 140 1 dry dimethylformamide was added 2.4 mg dicyclohexyl carbodiimide and 4.0 mg N-hydroxy succinimide. The reaction was incubated at room temperature for 12 hours, after which the crystals of dicyclohexyl urea formed were removed by centrifugation.

2 µl of this solution containing the activated ester of progesterone were then added to 1 mg of Aequorin dissolved in 200 1 of 65 mM phosphate buffer, pH 8.0. After 20 minutes incubation at room temperature, the progesterone-Aequorin conjugate was isolated by gel filtration on Sephadex G-25 in 50 mM Tris-HCl buffer, pH 7.4, containing 5 mM EDTA.

The progesterone-Aequorin conjugate was shown to be bioluminescent and immunologically active by its ability to react with solid-phase antiprogesterone IgG. See example 2, above. The conjugate was demonstrated to be stable over 4 months at -70°C and for greater than 2 weeks at 4°C. Equation (9) shows the synthesis of progesterone-aequorin conjugate.

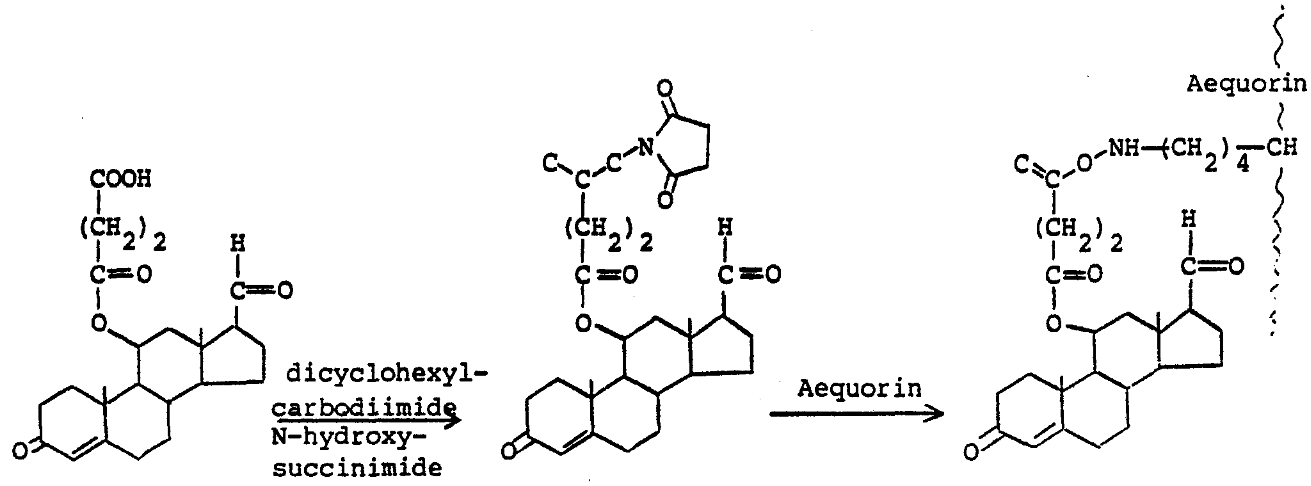


Progesterone-11α-
hemisuccinate

## Example 2

Progesterone-Aequorin Antibody Dilution Curve.

The progesterone-aequorin conjugate (50 µl; 1.7 x $10^{-14}$ mol, 3,300 total luminescence counts) was incubated for 2 hours with 50 µl buffer and 50 µl of varying dilutions of anti-progesterone IgG coupled to diazo-cellulose (solid-phase antibodies). The progesterone-aequorin conjugate bound to the solid-phase antibodies was separated by centrifugation and 100 µl aliquot of the supernatant assayed for bioluminescent activity. Bioluminescent activity was measured by injecting 20 µl of 0.2 M $CaCl_2$ into the aliquots in front of the photomultiplier tube and the total light produced in 15 seconds was measured.

The figure 1 shows the luminescence counts bound to the solid-phase antibodies at various dilutions in a progesterone-aequorin antibody dilution curve. Each point shows the mean ± S.D. of triplicate determinations.

## Example 3

Progesterone BLIA standard curve.

To the progesterone-aequorin conjugate (50 µl; 1.7 x $10^{-14}$ mol) was added 50 µl of 1/400 dilution of mid-phase antibodies (binding 50% of the conjugate, see figure 1 ) and 50 µl of varying concentrations of standard progesterone solutions (10 µM to 1 nm). After 2 hours incubation at room temperature, the amount of conjugate bound to the solid-phase antibodies was assayed as described in example 2, above.

Figure 2 shows the luminescence counts bound to the solid-phase antibodies at varying progesterone concentrations in a progesterone BLIA standard curve. Each point shows the mean ± S.D. of triplicate determinations.

## Example 4

Synthesis of Rabbit IgG-Aequorin Conjugate.

To 200 µg aequorin in 200µl of 100 mM phosphate buffer, pH 7.5 containing 10 mM EDTA (reaction buffer) was added 3.1 x $10^{-5}$ g of N-succinimidyl-3(2-pyridyldithio)-propionate (SDPD) in 2 µl of ethanol. After 30 min. incubation at room temperature, the aequorin containing 2-pyridyl disulphide moieties was isolated by gel filtration on Sephadex G-25.

Similarly, 1.5 mg rabbit IgG in 50 µl reaction buffer was reacted with 3.1 x $10^{-5}$g of SDPD and isolated in 100 mM Acetate buffer, pH 5.0, containing 0.15 M NaCl. The 2-pyridyl disulphide groups were then reduced to thiol groups with dithiothreotol (50 mM final concentration) for 20 minutes at room temperature. The excess reducing agent and pyridine-2-thione was removed by gel filtration and the thiol containing rabbit IgG was mixed with the aequorin containing the 2-pyridyl disulphide groups. After reacting

for 12 hours at room temperature, the unreacted aequorin and other side products were removed by ultra-filtration using an Amicon XM50 membrane. The rabbit IgG-aequorin conjugate was further purified by affinity chromatography using solid-phase sheep anti-rabbit IgG. The conjugate was demonstrated to be bioluminescent and immunologically active by ability to bind to the solid-phase antibodies. The conjugate was stable for 2 months when stored at -20°.

## Example 5

Rabbit IgG-aequorin antibody dilution curve.

50 µl of an appropriate dilution of the affinity purified rabbit IgG-aequorin conjugate (1,000 total luminescence counts) was incubated with 50 µl of varying dilutions of sheep and anti-rabbit IgG coupled to diazo--cellulose and 50 µl buffer. The amount of the rabbit IgG-aequorin conjugate bound to the solid-phase antibodies after a 2-hour incubation was quantified as described in example 2, above.

Figure 3 shows the luminescence counts (or rabbit IgG-aequorin conjugate) bound to the varying antibody dilutions in a rabbit IgG-aequorin conjugate binding curve. Each point represents the mean ± S.D. of triplicate determinations.

## Example 6

Synthesis of luciferase-antigen conjugate.

The heterobifunctional reagent, m-maleimidobenzyl-N--hydroxysuccinimide ester (MBS), is used to couple an antigen, through its free amino group, to the sulfhydryl group of Renilla luciferase as follows:

Dioxan (15 µl) containing 0.32 mg of MBS is added to 1.5 ml of phosphate buffer, pH 7.0, containing 1 mg of

antigen. The solution is maintained at 30°C for 1 hour and the MBS-reacted antigen is then desalted on Sephadex G-25. The MBS-reacted antigen is then mixed with 1 mg of Renilla luciferase at 4°C, allowed to incubate at 4°C for 6 hours, and the reaction was then terminated by the addition of ß-mercaptoethanol (10 mM, final concentration). Nonconjugated antigen was removed from labelled antigen by chromatography on Sephadex G-75. Free and bound label are separated by this procedure and easily monitored by taking column fraction aliquotes, adding luciferin (formula 1), and measuring light production.

Example 7

Homogeneous BLIA utilizing energy transfer.

To a luciferase-labelled antigen (see Example 6) is added an amount of GFP-labelled antibody which provides approximately 50% of the maximum observable energy transfer. The mixture is maintained in low ionic strength buffer such as mM Tris-HCl, pH 7-8. Upon the addition of luciferin (formula 1), a certain ratio of light emissions at 480 nm and at 509 nm occurs brought about by energy transfer as the result of interactions between the labelled antigen and GFP-labelled antibody. The simultaneous measure of both these light emissions is accomplished by a dual wavelength photometer. The ratio of light emission, i.e. 480/509 nm is recorded. As the amount of free antigen is increased in the reaction mixture, the ratio 480/509 nm increases due to a decrease in the amount of energy transfer.

Example 8

Homogeneous BLIA utilizing energy transfer and luciferin analogues.

The amounts of luciferase-labelled antigen and GFP-labelled antibody are the same as given in Example 7. The conditions are also the same. However, rather than initiating light production and energy transfer by the addition of luciferin (formula 1), an analogue of luciferin is used. For example, light production is initiated with a luciferin analogue where $R_1$ = phenyl and $R_3$= benzyl (formula 1). Due to energy transfer light emission is observed at 509 nm whereas little or no signal is observed at 480 nm. As the amount of free antigen is increased in the reaction mixture, the light intensity at 509 nm is decreased due to a decrease in energy transfer. In this example, only a single wavelength measurement is required to achieve a homogeneous BLIA.

## Example 9

An energy transfer-based homogeneous BLIA utilizing apophotoproteins and luciferin analogues.

The synthesis of an apophotoprotein-antigen conjugate may be achieved by following the procedure outlined in Example 1. The bound apophotoprotein is then converted to photoprotein-labelled antigen in the presence of an appropriate luciferin analogue by following the procedures outlined in equation 4. Low molecular weight components are then removed by gel filtration on G-25 Sephadex in EDTA--containing buffers. If a luciferin analogue, such as the one given in Example 8, is used during the recharging of the apophotoprotein, then little or no light is observed when $Ca^{2+}$ is added to the photoprotein-labelled antigen. However, when GFP-labelled antibody is added light emission at 509 nm is observed as the antigen-antibody complex is formed. To such a photoprotein-labelled antigen, is added an amount of GFP-labelled antibody which provides approximately 50% of the maximum observable signal at 509 nm upon the addition of $Ca^{2+}$. As the amount of free antigen is increased in the reaction mixture, the light intensity at 509 nm is decreased due to a decrease in energy transfer. This represents another example of a homogeneous BLIA by

measurement at a single wavelength.

The foregoing illustrates specific embodiments within the scope of this invention and is not to be construed as limiting said scope. While the invention has been described herein with regard to a certain specific embodiment, it is not so limited. It is to be understood that variations and modifications thereof may be made by those skilled in the art without departing from the scope of the invention.

CLAIMS

1. A protein-ligand conjugate suitable for use in a bioluminescence-linked binding assay, which comprises:

a ligand covalently attached to a protein selected from the group consisting of coelenterate-derived photoproteins, luciferases, apophotoproteins, and light energy transfer receptors.

2. The conjugate of Claim 1, wherein said ligand is an antigen or an antibody.

3. The conjugate of Claim 1, wherein said ligand is a hormone or a hormone receptor.

4. The conjugate of Claim 1, wherein said ligand is a nucleic acid.

5. The conjugate of Claim 1, wherein said protein is aequorin, obelin, mnemiopsin, or berovin or an apophotoprotein formed from aequorin, obelin, mnemiopsin, or berovin.

6. The conjugate of Claim 1, wherein said protein is green fluorescent protein.

7. A method for detecting a binding interaction between a first ligand and second ligand which comprises:

(a) providing said first ligand covalently attached to a protein label selected from the group consisting of coelenterate-derived photoproteins containing luciferin or a luciferin-like molecule, luciferases and apophotoproteins, which protein label is capable of being activated to cause emission of light,

(b) providing a second ligand covalently attached to a light energy transfer acceptor label capable of accepting light energy from a luciferin or a luciferin-like molecule and when complexed with the first ligand attached to said protein label is capable of modifying the light which the protein label causes to be emitted,

(c) contacting said first ligand with said second ligand to form a complex between said first and second ligand,

(d) contacting a light-triggering reagent with said complex, wherein

(1) when said label is a photoprotein containing a luciferin or luciferin-like molecule, calcium ions are contacted with said complex,

(2) when said label is a luciferase, a luciferin or luciferin-like molecule is contacted with said complex, and

(3) when said label is an apophotoprotein, calcium ions are contacted with said complex after said apophotoprotein is converted to a photoprotein by contact with a luciferin or luciferin-like molecule, and

(e) detecting light emitted by said complex.

8. A method for detecting a ligand in a sample through a binding interaction between a first ligand and second ligand which comprises:

(a) providing a first ligand which is covalently attached to a protein label selected from the group consisting of coelenterate-derived photoproteins containing luciferin or a luciferin-like molecule, luciferase and apophotoproteins, which protein label is capable of being activated to cause the emission of light,

(b) providing a second ligand covalently attached to a light energy transfer acceptor label capable of accepting light energy from a luciferin or a luciferin-like molecule and when complexed with the first ligand attached to said protein label is capable of modifying the light which the protein label causes to be emitted, either the first or the second ligand being the same as the ligand to be detected,

(c) contacting the sample with one of said first or second ligands which one ligand is not the same as the ligand to be determined in said sample and with a predetermined amount of the other of said first or second ligands to form a first complex between said one ligand which is not the same as the ligand to be determined in the sample with said ligand to be determined in the sample and a second complex between said one of said first or second ligands with the other ligand of said first or second ligands,

(d) contacting a light-triggering reagent with both of said complexes, wherein

(1) when said label is a photoprotein containing a luciferin or luciferin-like molecule, calcium ions are contacted with said complex,
(2) when said label is a luciferase, a luciferin or luciferine-like molecule is contacted with said complex, and
(3) when said label is an apophotoprotein calcium ion are contacted with said complex after said apophotoprotein is converted to a photoprotein by contact with a luciferin or luciferin-like molecule, and

(e) detecting light emitted by said luciferin or luciferin-like moleule.

9. A method for detecting a binding interaction between a first ligand and a second ligand, which comprises:

(a) contacting in a reaction mixture said first ligand with said second ligand wherein said second ligand is covalently attached to a protein label selected from the group consisting of coelenterate-derived photoproteins, luciferase, and apophotoproteins, whereby a bound complex is formed between said first ligand and said second ligand,

(b) separating said complex from the reaction mixture,

(c) contacting a light-triggering reagent with said complex to emit light, wherein

(1) when said label is a photoprotein containing a luciferin or luciferin-like molecule, calcium ions are contacted with said complex,
(2) when said label is a luciferase, a luciferin or luciferin-like molecule is contacted with said complex, and
(3) when said label is an apophotoprotein, calcium ions are contacted with said complex after said apophotoprotein is converted to a photoprotein by contact with a luciferin or luciferin-like molecule, and

(d) detecting light emitted by said complex.

10. A method for detecting a ligand in a sample through binding interaction between a first ligand and a second ligand, which comprises:

(a) contacting in a reaction medium said first ligand with a predetermined amount of said second ligand which is the same as the ligand to be detected wherein said second ligand is covalently attached to a protein label selected from the group consisting of coelenterate-derived photoproteins, luciferase, and apophotoproteins, and with the sample whereby a first complex is formed between said first ligand and said second ligand and a second complex is formed between said first ligand and the ligand to be detected

(b) separating from the reaction mixture both complexes,

(c) contacting a light-triggering reagent with both complexes to emit light, wherein

(1) when said label is a photoprotein containing a luciferin or luciferin-like molecule, calcium ions are contacted with both complexes,
(2) when said label is a luciferase, a luciferin or luciferin-like molecule is contacted with both complexes, and
(3) when said label is an apophotoprotein, calcium ions are contacted with both complexes after said apophotoprotein is converted to a photoprotein by contact with a luciferin or luciferin-like molecule, and

(d) detecting light emitted by said luciferin or luciferin-like molecule.

11. A method as claimed in any one of claims 7 to 11 wherein said binding interaction is an antigen-antibody, hapten-antibody, hormone-receptor, protein-nucleic acid, or nucleic acid-nuceic acid interaction.

12. A method as claimed in any one of claims 7 to 11, wherein said luciferin-like molecule has a formula:

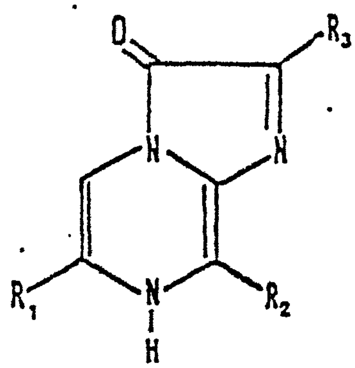

wherein $R_1$ is selected from the group consisting of an aromatic group, a heterocyclic group, an alkyl group, and hydrogen; wherein $R_2$ is selected from the group consisting of an aromatic group, a heterocyclic group, an alkyl group, and hydrogen; and wherein $R_3$ is selected from the group consisting of an aromatic group, a heterocyclic group, an alkyl group, and hydrogen.

13. A method as claimed in any one of claims 7 to 11, wherein said luciferin molecule has a formula:

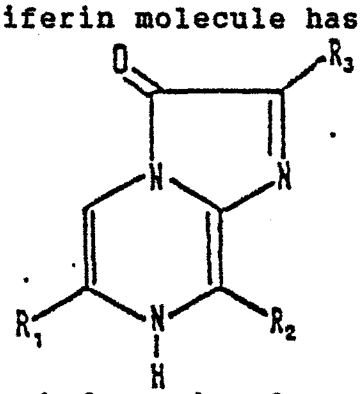

wherein $R_1$ is a p-hydroxyphenyl group, $R_2$ is a benzyl group, and $R_3$ is a p-hydroxybenzyl group.

14. A method as claimed in any one of claims 7 to 10, wherein said photoprotein is aequorin, obelin, mnemiopsin, or berovin.

15. A method as claimed in claim 7 or 8 wherein said second ligand is covalently attached to a light energy transfer acceptor label capable of accepting light energy from a luciferin or luciferin-like molecule.

16. A method as claimed in claim 7 or 8, wherein said light energy transfer acceptor is green fluorescent protein, fluorescein, phycoerythrin, rhodamine, a lucifer yellow dye, or 9,10-bisphenethynyl anthracene.

17. A kit suitable for conducting a bioluminescence--linked binding assay, which comprises:

a first container having confined therein a measured quantity of a first protein-ligand conjugate comprising a first ligand covalently attached to a protein label selected from the group consisting of coelenterate-derived photoproteins, luciferases, apophotoproteins, and light energy transfer receptors.

18. The kit of claim 17, wherein said protein is aequorin, obelin, mnemiopsin, or berovin or an apophotoprotein formed from aequorin, obelin, mnemiopsin, or berovin.

19. The kit of claim 17, wherein said first container contains a protein-ligand conjugate wherein the protein is a photoprotein, luciferase, or apophotoprotein and said kit further comprises a second container having confined therein a measured quantity of a second protein-ligand conjugate comprising a second ligand covalently attached to a light energy transfer receptor label, wherein said first ligand and said second ligand are capable of undergoing a binding interaction therebetween to form a bound complex.

20. The kit of claim 19 wherein said light energy transfer receptor label is green fluorescent protein.

***

CLAIMS for Austria

1. A method for detecting a binding interaction between a first ligand and second ligand which comprises:

(a) providing said first ligand covalently attached to a protein label selected from the group consisting of coelenterate-derived photoproteins containing luciferin or a luciferin-like molecule, luciferases and apophotoproteins, which protein label is capable of being activated to cause emission of light,

(b) providing a second ligand covalently attached to a light energy transfer acceptor label capable of accepting light energy from a luciferin or a luciferin-like molecule and when complexed with the first ligand attached to said protein label is capable of modifying the light which the protein label causes to be emitted,

(c) contacting said first ligand with said second ligand to form a complex between said first and second ligand,

(d) contacting a light-triggering reagent with said complex, wherein

(1) when said label is a photoprotein containing a luciferin or luciferin-like molecule, calcium ions are contacted with said complex,

(2) when said label is a luciferase, a luciferin or luciferin-like molecule is contacted with said complex, and

(3) when said label is an apophotoprotein, calcium ions are contacted with said complex after said apophotoprotein is converted to a photoprotein by contact with a luciferin or luciferin-like molecule, and

(e) detecting light emitted by said complex.

2. A method for detecting a ligand in a sample through a binding interaction between a first ligand and second ligand which comprises:

(a) providing a first ligand which is covalently attached to a protein label selected from the group consisting of coelenterate-derived photoproteins containing luciferin or a luciferin-like molecule, luciferase and apophotoproteins, which protein label is capable of being activated to cause the emission of light,

(b) providing a second ligand covalently attached to a light energy transfer acceptor label capable of accepting light energy from a luciferin or a luciferin-like molecule and when complexed with the first ligand attached to said protein label is capable of modifying the light which the protein label causes to be emitted, either the first or the second ligand being the same as the ligand to be detected,

(c) contacting the sample with one of said first or second ligands which one ligand is not the same as the ligand to be determined in said sample and with a predetermined amount of the other of said first or second ligands to form a first complex between said one ligand which is not the same as the ligand to be determined in the sample with said ligand to be determined in the sample and a second complex between said one of said first or second ligands with the other ligand of said first or second ligands,

(d) contacting a light-triggering reagent with both of said complexes, wherein

(1) when said label is a photoprotein containing a luciferin or luciferin-like molecule, calcium ions

are contacted with said complex,
(2) when said label is a luciferase, a luciferin or luciferine-like molecule is contacted with said complex, and
(3) when said label is an apophotoprotein calcium ion are contacted with said complex after said apophotoprotein is converted to a photoprotein by contact with a luciferin or luciferin-like molecule, and

(e) detecting light emitted by said luciferin or luciferin-like moleule.

3. A method for detecting a binding interaction between a first ligand and a second ligand, which comprises:

(a) contacting in a reaction mixture said first ligand with said second ligand wherein said second ligand is covalently attached to a protein label selected from the group consisting of coelenterate-derived photoproteins, luciferase, and apophotoproteins, whereby a bound complex is formed between said first ligand and said second ligand,

(b) separating said complex from the reaction mixture,

(c) contacting a light-triggering reagent with said complex to emit light, wherein

(1) when said label is a photoprotein containing a luciferin or luciferin-like molecule, calcium ions are contacted with said complex,
(2) when said label is a luciferase, a luciferin or luciferin-like molecule is contacted with said complex, and
(3) when said label is an apophotoprotein, calcium ions are contacted with said complex after said apophotoprotein is converted to a photoprotein by

contact with a luciferin or luciferin-like molecule, and

(d) detecting light emitted by said complex.

4. A method for detecting a ligand in a sample through binding interaction between a first ligand and a second ligand, which comprises:

(a) contacting in a reaction medium said first ligand with a predetermined amount of said second ligand which is the same as the ligand to be detected wherein said second ligand is covalently attached to a protein label selected from the group consisting of coelenterate-derived photoproteins, luciferase, and apophotoproteins, and with the sample whereby a first complex is formed between said first ligand and said second ligand and a second complex is formed between said first ligand and the ligand to be detected

(b) separating from the reaction mixture both complexes,

(c) contacting a light-triggering reagent with both complexes to emit light, wherein

(1) when said label is a photoprotein containing a luciferin or luciferin-like molecule, calcium ions are contacted with both complexes,
(2) when said label is a luciferase, a luciferin or luciferin-like molecule is contacted with both complexes, and
(3) when said label is an apophotoprotein, calcium ions are contacted with both complexes after said apophotoprotein is converted to a photoprotein by contact with a luciferin or luciferin-like molecule, and

(d) detecting light emitted by said luciferin or luciferin-like molecule.

5. A method as claimed in any one of claims 1 to 4 wherein said binding interaction is an antigen-antibody, hapten-antibody, hormone-receptor, protein-nucleic acid, or nucleic acid-nuceic acid interaction.

6. A method as claimed in any one of claims 1 to 5, wherein said luciferin-like molecule has a formula:

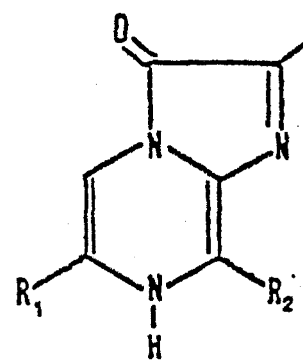

wherein $R_1$ is selected from the group consisting of an aromatic group, a heterocyclic group, an alkyl group, and hydrogen; wherein $R_2$ is selected from the group consisting of an aromatic group, a heterocyclic group, an alkyl group, and hydrogen; and wherein $R_3$ is selected from the group consisting of an aromatic group, a heterocyclic group, an alkyl group, and hydrogen.

7. A method as claimed in any one of claims 1 to 5, wherein said luciferin molecule has a formula:

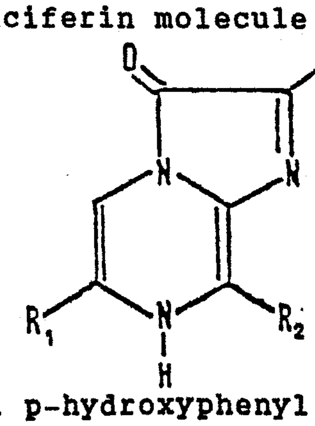

wherein $R_1$ is a p-hydroxyphenyl group, $R_2$ is a benzyl

group, and $R_3$ is a p-hydroxybenzyl group.

8. A method as claimed in any one of claims 1 to 4, wherein said photoprotein is aequorin, obelin, mnemiopsin, or berovin.

9. A method as claimed in claim 1 or 2, wherein said second ligand is covalently attached to a light energy transfer acceptor label capable of accepting light energy from a luciferin or luciferin-like molecule.

10. A method as claimed in claim 1 or 2, wherein said light energy transfer acceptor is green fluorescent protein, fluorescein, phycoerythrin, rhodamine, a lucifer yellow dye, or 9,10-bisphenethynyl anthracene.

11. A kit suitable for conducting a bioluminescence--linked binding assay, which comprises;

a first container having confined therein a measured quantity of a first protein-ligand conjugate comprising a first ligand covalently attached to a protein label selected from the group consisting of coelenterate-derived photoproteins, luciferases, apophotoproteins, and light energy transfer receptors.

12. The kit of claim 11, wherein said protein is aequorin, obelin, mnemiopsin, or berovin or an apophotoprotein formed from aequorin, obelin, mnemiopsin, or berovin.

13. The kit of claim 11, wherein said first container contains a protein-ligand conjugate wherein the protein is a photoprotein, luciferase, or apophotoprotein and said kit further comprises a second container having confined therein a measured quantity of a second protein-ligand conjugate comprising a second ligand covalently attached to a light energy transfer receptor label, wherein said first

ligand and said second ligand are capable of undergoing a binding interaction therebetween to form a bound complex.

14. The kit of claim 13 wherein said light energy transfer receptor label is green fluorescent protein.

***

0137515

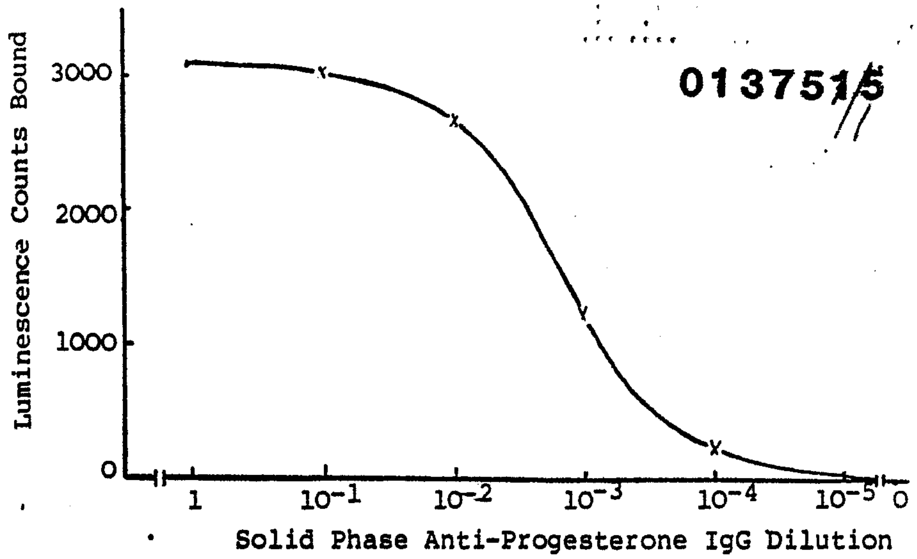

Figure 1

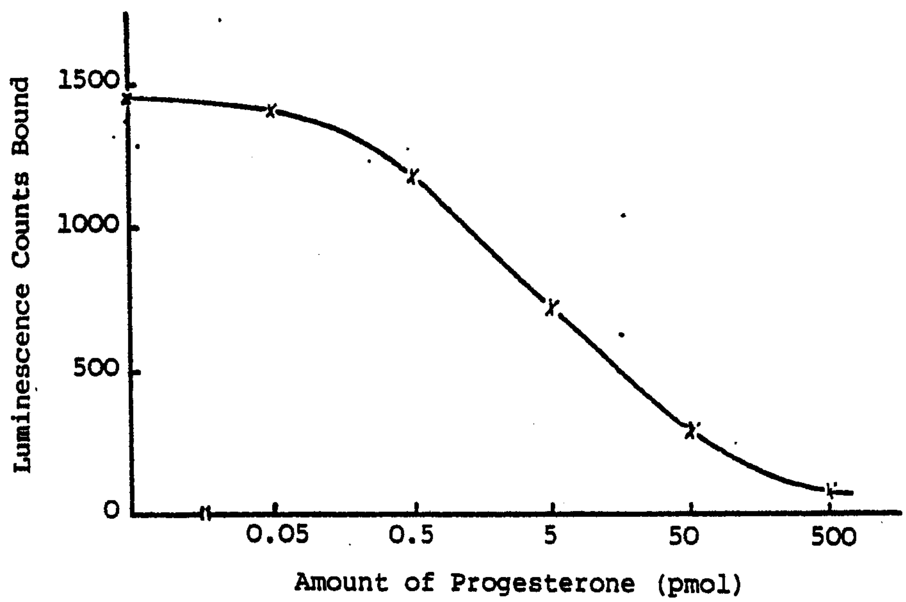

Figure 2

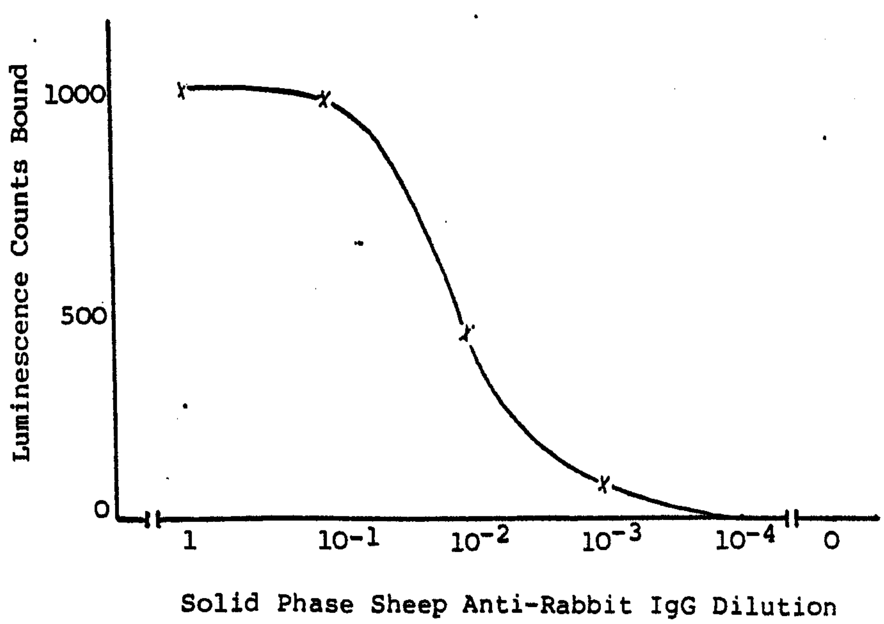

Figure 3